# EUROPÄISCHE PATENTSCHRIFT

(11) **EP 4 687 718 B1**
(45) Veröffentlichungstag und Bekanntmachung des Hinweises auf die Patenterteilung: **30.09.2026**
(21) Anmeldenummer: 24703126.3
(22) Anmeldetag: 30.01.2024
(51) Int. Cl.: A61B 17/70

(54) **KNOCHENANKER, INSBESONDERE KNOCHENSCHRAUBE, INSBESONDERE PEDIKELSCHRAUBE**
BONE ANCHOR, IN PARTICULAR BONE SCREW, IN PARTICULAR PEDICLE SCREW
ANCRAGE OSSEUX, EN PARTICULIER VIS À OS, EN PARTICULIER VIS PÉDICULAIRE

(30) Priorität: 29.03.2023 DE 102023107971
(43) Veröffentlichungstag der Anmeldung: 11.02.2026
(73) Patentinhaber: Silony Medical International AG, 8500 Frauenfeld (CH)
(72) Erfinder: WECKLER, David, 73274 Notzingen (DE); VENZIN, Raphael, 71229 Leonberg (DE); KLABUNDE, Ralf, 8405 Winterthur (CH)
(74) Vertreter: DREISS Patentanwälte PartG mbB
(86) Internationale Anmeldenummer: PCT/EP2024/052167
(87) Internationale Veröffentlichungsnummer: WO 2024/199761

(56) Entgegenhaltungen:
- EP-B1- 2 560 564
- US-A1- 2015 066 042
- US-A1- 2017 164 980
- US-A1- 2022 249 136
- US-B2- 8 764 806

## Beschreibung

Die Erfindung betrifft einen Knochenanker, insbesondere Knochenschraube, insbesondere Pedikelschraube, insbesondere für die minimalinvasive Wirbelsäulenchirurgie, wobei der Knochenanker einen in einen Knochen einsetzbaren Schaft und einen gegenüber dem Schaft polyaxial orientierbaren Gabelkopf mit zwei Schenkeln zur Aufnahme eines Korrekturstabs umfasst, wobei der Gabelkopf eine axiale Längsrichtung, eine hierzu konzentrische Umfangsrichtung und eine radiale Richtung definiert, mit einer Verlängerungsvorrichtung, die eine Zugangsöffnung zu dem Gabelkopf und zu dem Schaft bildet und die winkelstabil an dem Gabelkopf lösbar befestigbar ist, wobei eine vom Chirurgen gewünschte Orientierung des Gabelkopfs gegenüber dem Schaft temporär, das heißt lösbar festlegbar ist, um währenddessen Korrekturmaßnahmen bei dem Knochen ausführen zu können.

Knochenanker der hier in Rede stehenden Art werden typischerweise bei der minimalinvasiven Wirbelsäulenchirurgie verwendet, wobei die während des operativen Eingriffs am Gabelkopf des Knochenankers festgelegte Verlängerungsvorrichtung eine tunnelförmige Zugangsöffnung zu dem Schaft und zu dem Gabelkopf des Knochenankers bildet oder zumindest teilweise begrenzt. Durch diese Zugangsöffnung hindurch kann dann mittels eines Instruments mit langgestrecktem Instrumentenschaft, etwa mittels eines Schraubendrehers, Zugang zu dem Schaft des Knochenankers oder zu einer in den Gabelkopf einzuschraubenden Madenschraube genommen werden oder es kann Zugang zu dem Knochenanker zur Ausführung sonstiger Maßnahmen oder zur Einbringung von Implantaten genommen werden. Zur Bildung der Verlängerungsvorrichtung können Verlängerungshülsen oder ein Schalenteil oder insbesondere mehrere Schalenteile, insbesondere Halbschalenteile, verwendet werden, die für die Dauer des operativen Eingriffs lösbar am Gabelkopf des Knochenankers festlegbar sind oder die herstellerseitig an den Gabelkopf angefügt und anschließend hiervon abtrennbar sind.

Im Zuge von Wirbelsäulenoperationen und insbesondere von Fusionsmaßnahmen bei Wirbelkörpern der Wirbelsäule sind mitunter Korrekturmaßnahmen bei Knochen oder speziell bei Wirbelkörpern erforderlich. Diese werden durch den Chirurgen durch Einleitung von Kräften und Momenten in den Knochen über den in den Knochen oder Wirbelkörper eingeschraubten Knochenanker und/oder dessen Verlängerungsvorrichtung ausgeführt. Dies geschieht unter Verwendung einer sogenannten retraktierenden Komponente, die häufig als Retraktor oder Retraktorvorrichtung bezeichnet wird, im einfachsten Fall aber aus einer zangenförmigen Komponente bestehen kann. Hierdurch können korrigierende oder repositionierende Veränderungen bei dem Knochen oder Wirbelkörper ausgeführt werden, insbesondere Distraktion, Kompression, Lordosierung oder Kyphosierung von Knochen oder Wirbelkörpern zueinander.

US 8,764,806 B2 zeigt einen Knochenanker mit Verlängerungsvorrichtung. Die Verlängerungsvorrichtung umfasst eine äußere Schraubhülse, die über einen Stab gestülpt wird und dann mitsamt des innenliegenden Stabs am Gabelkopf festgeschraubt wird, wobei der innenliegende Stab mit einem Außensechskant an seinem gabelkopfseitigen Ende in einen Innensechskant des Schafts drehfest eingreift, so dass mittels der festgeschraubten Verlängerungsvorrichtung und mittels des Stabs der Gabelkopf und der Schaft in nur einer einzigen fluchtenden Längsorientierung zueinander lösbar und drehfest zueinander festgelegt sind. In dieser Anordnung ist der Knochenanker mit Gabelkopf und Schaft drehbar, und der Schaft kann in einen Knochen einschraubt werden. Der innenliegende Stab erstreckt sich hierfür körperabgewandt aus der Verlängerungsvorrichtung heraus und weist dort eine Werkzeugansetzstelle in Form eines Außensechskants auf. Er trägt dort außerdem einen Außengewindeabschnitt; dort lässt sich eine weitere Vorrichtung zur Ausführung einer Distraktion oder Kompression in Bezug auf benachbarte Knochenanker bzw. Wirbel mittels einer Schraubenmutter befestigen, wodurch aber keine weitergehende Klemmkraft auf den innenliegenden Stab oder den Schaft ausgeübt wird.

Der vorliegenden Erfindung liegt die Aufgabe zugrunde, den Knochenanker dahingehend auszubilden, dass solche Korrekturmaßnahmen noch handhabungsfreundlicher und vor allem sicherer ausführbar sind.

Diese Aufgabe wird bei einem Knochenanker der eingangsgenannten Art erfindungsgemäß dadurch gelöst, dass der Knochenanker weiterhin ein Klemmstück umfasst, das zum temporären Festlegen der Orientierung des Gabelkopfs gegenüber dem Schaft durch die Zugangsöffnung hindurch dem Gabelkopf zuführbar ist und dort unmittelbar oder mittelbar gegen den Schaft klemmbar ist, indem auf das Klemmstück eine temporäre Stellkraft in der axialen Längsrichtung ausübbar ist, und dass der Knochenanker weiterhin ein Stellmittel umfasst und dass die temporäre Stellkraft in der axialen Längsrichtung durch Abstützung des Stellmittels gegen die Verlängerungsvorrichtung erzeugbar und in das Klemmstück einleitbar ist, und dass sich das Klemmstück dabei in radialer Richtung zwischen die Schenkel des Gabelkopfs erstreckt und somit gegenüber dem Gabelkopf in Umfangsrichtung abgestützt und unverdrehbar ist.

Es wird also erfindungsgemäß vorgeschlagen, dass zum temporären Festlegen der Orientierung des Gabelkopfs gegenüber dem Schaft ein Klemmstück zugeführt und in einer bestimmungsgemäßen Position im Gabelkopf in Richtung der axialen Längsrichtung mit einer Stellkraft belastet wird, welche durch Abstützung gegenüber der am Gabelkopf bereits festgelegten Verlängerungsvorrichtung erzeugt ist. Hierdurch lässt sich eine noch weitergehende Verspannung und Fixierung der Verlängerungsvorrichtung am Gabelkopf erreichen. Wenn diese Abstützung eines hierfür verwandten Stellmittels gegen die Verlängerungsvorrichtung verhältnismäßig nahe am gabelkopfseitigen Ende realisiert ist, so lässt sich ein hierfür verwendetes Instrument wieder aus der Verlängerungsvorrichtung entnehmen, so dass die von der Verlängerungsvorrichtung begrenzte langgestreckte und insbesondere tunnelförmige Zugangsöffnung frei ist und nach oben keine störenden Komponenten aus der Verlängerungsvorrichtung hervorstehen. Des Weiteren wird vorgeschlagen, dass sich das eingebrachte Klemmstück in radialer Richtung zwischen die Schenkel des Gabelkopfs erstreckt, wodurch eine bestimmungsgemäße Betriebsposition vorgegeben sein kann und das Klemmstück in jedem Fall unverdrehbar bezüglich des Gabelkopfs gestellt ist. Beides erweist sich zum Herbeiführen einer temporären Klemmung gegenüber dem Ankerschaft zum temporären Festlegen der Orientierung des Gabelkopfs gegenüber dem Ankerschaft als vorteilhaft. In diesem temporär festgelegten Zustand lassen sich mittels der genannten retraktierenden Komponente Korrekturmaßnahmen ausführen. Nach dem Lösen dieser temporären Festlegung der Orientierung des Gabelkopfs, also nach dem Lösen oder Wegnehmen der temporären Stellkraft auf das Klemmstück, lässt sich das Klemmstück wieder aus der Zugangsöffnung entnehmen, um dann schließlich einen Korrekturstab durch die Zugangsöffnung einzuführen und am Gabelkopf in an sich bekannter Weise, insbesondere durch Festziehen einer Madenschraube, gegenüber einem Innengewinde des Gabelkopfes zu fixieren.

Bei einer bevorzugten Ausführungsform des Knochenankers und dessen Klemmstücks wird vorgeschlagen, dass das Klemmstück einen Auflagerabschnitt aufweist, mit dem es mittelbar oder unmittelbar gegen den Schaft anlegbar ist und dass sich das Adapterteil dabei insbesondere mit diesem Auflagerabschnitt in der bestimmten radialen Richtung über die Kernöffnung nach außen erstreckt (Anspruch 2). Dieser Auflagerabschnitt ist vorzugsweise komplementär zu einem in den Gabelkopf ragenden Ende oder Kopf des Ankerschafts oder komplementär zu einem dort eingelegten Zwischendruckstück ausgebildet, wobei das Zwischendruckstück die Aufgabe hat, den Gabelkopf zwar vorzugsweise polyaxial verschwenkbar, jedoch gleichwohl unverlierbar, am Ankerschaft zu halten und die temporäre Stellkraft gleichmäßig in den Ankerschaft bzw. den Kopf des Ankerschafts des Knochenankers einzuleiten.

Wie erwähnt, wird die temporäre Stellkraft in der axialen Längsrichtung durch Abstützen des Stellmittels gegen die Verlängerungsvorrichtung erzeugt. Das Stellmittel kann also einenends in einer geeigneten Weise innen und/oder außen axial gegenüber der Verlängerungsvorrichtung abgestützt sein und sich anderenends gegen das Klemmstück oder dessen Auflagerabschnitt abstützen, um die Stellkraft in der axialen Längsrichtung auf den Anker zu übertragen. Hierfür wäre grundsätzlich ein sich durch die Verlängerungsvorrichtung hindurcherstreckender Instrumententschaft mit einem Kniehebelmechanismus oder mit einer Gewindeverbindung zu der Verlängerungsvorrichtung denkbar. Nach einer bevorzugten Ausführungsform erweist es sich als vorteilhaft, wenn das Stellmittel einen Gewindeträgerabschnitt mit einem Außengewinde aufweist, und die Verlängerungsvorrichtung ein Innengewinde aufweist, in welches das Außengewinde des Gewindeträgerabschnitts einschraubbar ist, so dass hierdurch die temporäre Stellkraft in der axialen Längsrichtung über das Klemmstück auf den Schaft ausübbar ist, um die Orientierung des Gabelkopfs gegenüber dem Schaft temporär und lösbar zu fixieren (Anspruch 3).

Dieser Gewindeträgerabschnitt kann in der axialen Längsrichtung insbesondere kurz bauen, insbesondere nur 5 bis 25 mm lang sein, wobei dessen Außengewinde in der Längsrichtung nur über wenige Millimeter erstreckt sein kann, insbesondere 2-6 mm, insbesondere 3-6 mm sind hinreichend. Solchenfalls ist auch das Innengewinde an der Verlängerungsvorrichtung verhältnismäßig nahe am Gabelkopf angeordnet. Der Gewindeträgerabschnitt des Stellmittels lässt sich mittels eines in die Verlängerungsvorrichtung einführbaren Schraubendreherinstruments in das Innengewinde bei der Verlängerungsvorrichtung einschrauben und festziehen. Wenn das Schraubendreherinstrument aus der Verlängerungsöffnung herausgezogen ist, so ist diese wieder frei zugänglich, und es stehen auch keine störenden Komponenten aus der Verlängerungsvorrichtung hervor.

Der Gewindeträgerabschnitt kann als von dem Klemmstück oder dessen Auflagerabschnitt separates Teil ausgebildet sein. Es erweist sich indessen als vorteilhaft, wenn das Stellmittel, insbesondere der Gewindeträgerabschnitt, unverlierbar mit dem Klemmstück angeordnet ist. Es lässt sich so beim Zuführen und beim Entnehmen als einheitliche Baugruppe handhaben (Anspruch 4).

Weiter kann es sich als vorteilhaft erweisen, wenn das Stellmittel, insbesondere der Gewindeträgerabschnitt, insbesondere nur geringfügig, das heißt einige wenige mm, axial verschieblich an dem Klemmstück und insbesondere an dem Auflagerabschnitt geführt und vorzugsweise drehbar an dem Klemmstück und insbesondere an dem Auflagerabschnitt des Klemmstücks gehalten ist. Durch diese Maßnahmen lässt sich eine bestimmungsgemäße vorgegebene Orientierung des Stellmittels zu dem Klemmstück realisieren (Anspruch 5).

In Weiterbildung dieses Gedankens erweist es sich als vorteilhaft, wenn der Gewindeträgerabschnitt auf einem in der axialen Längsrichtung erstreckten und an dem Klemmstück, insbesondere an dem Auflagerabschnitt, gehaltenen Stift beschränkt verschieblich und drehbar geführt ist. Hierfür kann es sich als vorteilhaft erweisen, wenn der Stift im Bereich seines Endes ein Außengewinde trägt, welches an einen gewindelosen Stiftabschnitt angrenzt (Anspruch 6). Der Gewindeträgerabschnitt lässt sich dann mit einer zentralen Bohrung mit einem kurzen Innengewindeabschnitt auf das Außengewinde des Stifts aufschrauben bis das Außengewinde des Stifts in eine größere gewindelose Öffnung eingreift und der Gewindeträgerabschnitt dadurch gegenüber dem Klemmstück beschränkt axial verschieblich wird und dabei unverlierbar an dem Klemmstück angeordnet ist.

Wie eingangs bereits erwähnt, kann es sich als vorteilhaft erweisen, wenn in den Gabelkopf ein Zwischenstück oder Zwischendruckstück eingesetzt ist, welches zwischen einem Kopf des Schafts und einem in den Gabelkopf einlegbaren und dort dauerhaft fixierbaren Korrekturstab oder dem Auflagerabschnitt des Klemmstücks angeordnet ist, und wenn der Auflagerabschnitt des Druckstücks auf seiner dem Zwischenstück zugewandten Seite komplementär zu dem Zwischenstück ausgebildet ist (Anspruch 7). Zweckmäßigerweise ist das Zwischenstück komplementär zum Außenumfang eines in den Gabelkopf einsetzbaren Korrekturstabs ausgebildet, und entsprechend ist dann das Klemmstück oder der Auflagerabschnitt des Klemmstücks entsprechend dem Außenumfang des Korrekturstabs ausgebildet.

Gerade im Zusammenwirken mit einem Zwischenstück zwischen dem Schaft und dem Klemmstück erweist es sich als vorteilhaft, dass das Klemmstück verdrehsicher im Gabelkopf gehalten ist, so dass es keine das Zwischenstück verwindenden oder verbiegenden Kräfte auf dieses überträgt.

Wie eingangs ebenfalls ausgeführt, kann die Verlängerungsvorrichtung als starres, verwindungssteifes, d.h. Kraft und Drehmoment aufnehmendes, Hülsenteil oder Schalenteil ausgebildet sein (Anspruch 8). Nach einer Ausführungsform der Erfindung erweist es sich als vorteilhaft, wenn die Verlängerungsvorrichtung ein einziges starres, verwindungssteifes Hülsenteil oder Schalenteil umfasst, welches an seinem dem Gabelkopf zugewandten Ende zwei voneinander beabstandete und durch einen in der axialen Längsrichtung ausmündenden Trennschlitz voneinander getrennte Befestigungsabschnitte aufweist, mittels derer das Hülsenteil oder Schalenteil an den Schenkeln des Gabelkopfs lösbar festlegbar ist (Anspruch 9).

Es ist weiter vorteilhaft und ausreichend, wenn das Innengewinde an der Verlängerungsvorrichtung eine Erstreckung in der Umfangsrichtung von wenigstens 180°, insbesondere von wenigstens 181°, insbesondere von wenigstens 182°, insbesondere von wenigstens 185° und insbesondere von höchstens 200°, insbesondere von höchstens 195°, insbesondere von höchstens 190° aufweist (Anspruch 10). Im Zusammenhang mit einer halboffenen Ausgestaltung der Verlängerungsvorrichtung, als halboffene Hülse oder als einziges Schalenteil, ist eine derartige Umfangserstreckung des Gewindes ausreichend, um das Stellmittel bzw. dessen Gewindeträgerabschnitt verliersicher in dieses Gewinde einzuschrauben. Weiter ist es durch die solchermaßen offene Ausgestaltung der Verlängerungsvorrichtung leicht möglich, einen durch die Verlängerungsvorrichtung hindurchgeführten Korrekturstab in seine bestimmungsgemäße Fixierposition im Gabelkopf zu bringen.

Zum Festlegen der Verlängerungsvorrichtung am Gabelkopf wird weiter vorgeschlagen, dass die Verlängerungsvorrichtung in eine Hintergriffsstellung am Gabelkopf drehbar ist. Solchenfalls ist am Gabelkopf ein hintergreifbarer Bereich, vorzugsweise in Form einer die radiale Richtung hintergreifenden hakenförmigen Nut ausgebildet, in welche die Verlängerungsvorrichtung mit einem hierzu komplementär ausgebildeten Eingriffsbereich eindrehbar ist (Anspruch 11).

In Weiterbildung dieses Gedankens erweist es sich als vorteilhaft, wenn die Verlängerungsvorrichtung in der Hintergriffsstellung gegen Rückdrehen sicherbar ist (Anspruch 12). Hierfür wird vorgeschlagen, dass ein Sicherungsmittel in Form eines in der axialen Längsrichtung verschieblichen Sperrorgans vorzusehen, insbesondere in Form eines Stifts, das in der Verlängerungsvorrichtung geführt ist und sich in Umfangsrichtung gegen einen Schenkel des Gabelkopfs abstützt und hierdurch ein unbeabsichtigtes Rückdrehen der Verlängerungsvorrichtung verhindert (Anspruch 13). Um eine gute Zugänglichkeit zu dem betätigbaren Sperrorgan zu gewährleisten, wird vorgeschlagen, dass sich das Sperrorgan in der axialen Längsrichtung bis zu einem vom Gabelkopf abgewandten Ende der Verlängerungsvorrichtung erstreckt und dort zur Ausführung einer Stellbewegung zugänglich ist (Anspruch 14).

Es erweist sich weiter als vorteilhaft, wenn die Verlängerungsvorrichtung in der Hintergriffsstellung gegen einen in der Umfangsrichtung wirkenden Anschlag am Gabelkopf anlegbar ist. Im Ergebnis kann die Verlängerungsvorrichtung in der einen Umfangsrichtung gegen den genannten Anschlag am Gabelkopf anliegen und in der Gegenrichtung gegen das Sperrorgan. Die Orientierung des Anschlags wird dann vorteilhafterweise dahingehend gewählt, dass die Verlängerungsvorrichtung eine Gegenhalterfunktion, also eine stützende Funktion des Gabelkopfs beim finalen Festsetzen der Klemmung des Korrekturstabs im Gabelkopf ausübt (Anspruch 15).

Nach einem weiteren Gedanken von an sich eigenständiger Bedeutung wird ein Knochenanker der vorausgehend beschriebenen Art oder ein Knochenanker lediglich mit den Oberbegriffsmerkmalen des Anspruchs 1 vorgeschlagen, welcher gekennzeichnet ist durch ein Verbindungsteil als Schnittstelle zwischen der am Gabelkopf festgelegten Verlängerungsvorrichtung und eine retraktierenden Komponente oder Retraktorvorrichtung, wobei das Verbindungsteil lösbar an der Verlängerungsvorrichtung anordenbar ist (Anspruch 16). In Weiterbildung dieses Gedankens wird vorgeschlagen, dass das Verbindungsteil in der axialen Längsrichtung lösbar auf die Verlängerungsvorrichtung aufschiebbar oder jedenfalls an der Verlängerungsvorrichtung verschieblich ist und hierfür ein in der axialen Längsrichtung erstrecktes Längsführungsmittel an der Verlängerungsvorrichtung und an dem Verbindungsteil vorgesehen ist (Anspruch 17). Das aufschiebbare Verbindungsteil kann nach Gebrauch vorzugsweise wieder abgenommen werden, insbesondere abgezogen werden. Weiter wird diesbezüglich vorgeschlagen, dass das Längsführungsmittel formschlüssig wirkend ist und insbesondere nut- und federartig, insbesondere wenigstens halbseitig schwalbenschwanzförmig, ausgebildet ist (Anspruch 18).

Im Hinblick auf eine kompakte Bauform erweist es sich als vorteilhaft, wenn das Verbindungsteil einen schalenförmigen und komplementär zu der Verlängerungsvorrichtung ausgebildeten Abschnitt aufweist. Auf diese Weise können sich das Verbindungsteil und die Verlängerungsvorrichtung ungefähr konzentrisch zueinander erstrecken (Anspruch 19).

Zur Kopplung einer oder mehrerer Verbindungsvorrichtungen mit einer retraktierenden Komponente erweist es sich als vorteilhaft, wenn das Verbindungsteil einen quer zur axialen Längsrichtung nach außen erstreckten Befestigungsabschnitt zur lösbaren formschlüssigen Verbindung und Fixierung mit einer retraktierenden Komponente (Retraktorvorrichtung) aufweist (Anspruch 20).

Weitere Einzelheiten, Merkmale und Vorteile der Erfindung ergeben sich aus den beigefügten Patentansprüchen und aus der zeichnerischen Darstellung und nachfolgenden Beschreibung einer bevorzugten Ausführungsform des erfindungsgemäßen Knochenankers.

In der Zeichnung zeigt:
- Figur 1: eine perspektivische Ansicht einer Ausführungsform des erfindungsgemäßen Knochenankers mit Verlängerungsvorrichtung;

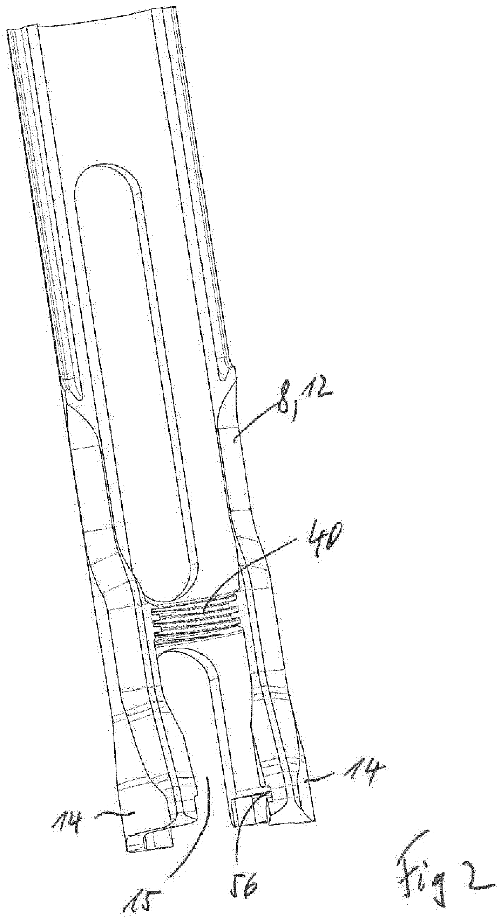

- Figuren 2, 3: perspektivische Ansichten der Verlängerungsvorrichtung von Figur 1;

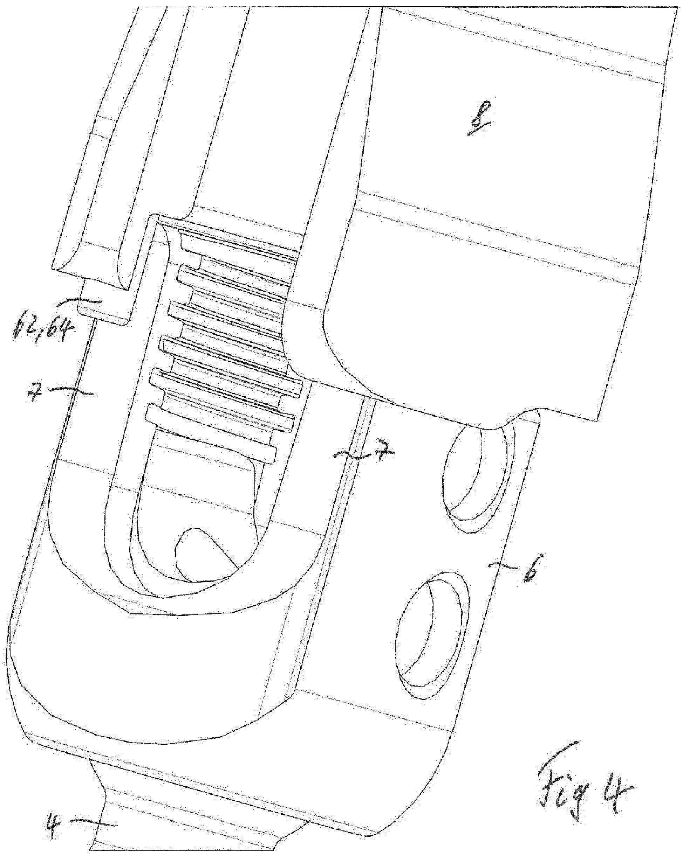

- Figur 4: eine vergrößerte Detailansicht des Knochenankers nach Figur 1 im Verbindungsbereich zwischen Verlängerungsvorrichtung und Gabelkopf;
- Figuren 5a-c: perspektivische Ansichten eines Klemmstücks des erfindungsgemäßen Knochenankers;
- Figur 6: eine teilweise perspektivische Ansicht des erfindungsgemäßen Knochenankers im erfindungsgemäß temporär fixierten Zustand unter Verwendung des Klemmstücks;
- Figuren 7a-d: verdeutlichen die Befestigung der Verlängerungsvorrichtung am Gabelkopf;
- Figuren 8, 9: perspektivische Ansichten eines Verbindungsteils zur Verbindung der Verlängerungsvorrichtung mit einer retraktierenden Komponente;
- Figuren 10, 11: verdeutlichen das Aufschieben des Verbindungsteils auf die Verlängerungsvorrichtung; und
- Figur 12: zeigt schematisch eine retraktierende Komponente in einer Betriebsverbindung an zwei benachbarten erfindungsgemäßen Knochenankern.

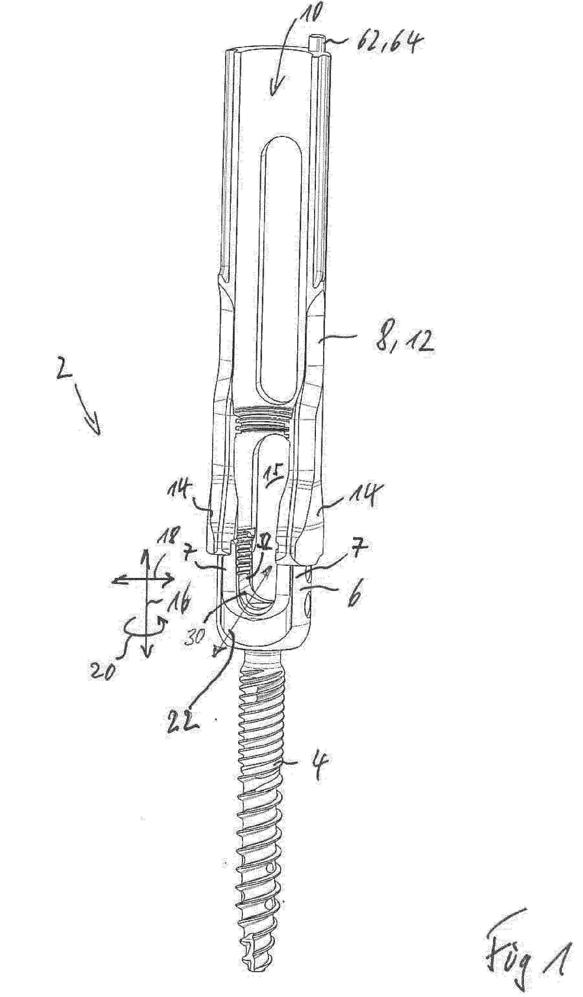

Die Figuren 1 und 6 sowie 10-12 zeigen perspektivische Ansichten des insgesamt mit dem Bezugszeichen 2 bezeichneten Knochenankers. Der hier beispielhaft als Pedikelschraube für die minimalinvasive Wirbelsäulenchirurgie ausgebildete Knochenanker 2 umfasst einen in einen Knochen, insbesondere in das Pedikel eines Wirbelkörpers, einsetzbaren Schaft 4 und einen den Schaft 4 zunächst polyaxial verschwenkbar aufnehmenden und haltenden Gabelkopf 6 mit zwei Schenkeln 7 und eine Verlängerungsvorrichtung 8, die eine im Wesentlichen tunnelförmige jedoch nur halbseitig begrenzte Zugangsöffnung 10 zu dem Gabelkopf 6 und dem darin gehaltenen Schaft 4 bildet. Die noch näher zu erläuternde Verlängerungsvorrichtung 8 umfasst im beispielhaft dargestellten Fall ein Schalenteil 12, welches sich etwas mehr als 180° in einer Umfangsrichtung erstreckt. Das Schalenteil 12 umfasst zum Gabelkopf 6 hin zwei Befestigungsabschnitte 14, die durch einen Schlitz 15 voneinander getrennt sind und mittels derer das Schalenteil 12 lösbar am Gabelkopf 6 winkelstabil befestigt werden kann, um während des chirurgischen Eingriffs die schon erwähnte tunnelförmige Zugangsöffnung 10 zu bilden, damit der Eingriff minimalinvasiv ausgeführt werden kann. Durch die Zugangsöffnung 10 hindurch lässt sich während des chirurgischen Eingriffs ein nicht dargestellter Korrekturstab an den bereits an der Wirbelsäule verankerten Knochenanker 2 heranführen und im Gabelkopf 6 des Knochenankers dauerhaft auf an sich bekannte Weise fixieren, so dass benachbarte Knochenanker mittels des Korrekturstabs miteinander verbunden und fixiert werden können, indem der Korrekturstab am jeweiligen Gabelkopf 6 benachbarter Knochenanker 2 dauerhaft fixiert wird (Wirbelkörperfusion). Hiernach lässt sich das Schalenteil 12 und damit die Verlängerungsvorrichtung 8 vom Gabelkopf 6 wieder ablösen und aus der Operationsöffnung entnehmen.

Der Gabelkopf 6 des Knochenankers 2 definiert eine axiale Längsrichtung 16 und eine hierzu radiale Richtung 18 und eine zur axialen Längsrichtung 16 konzentrische Umfangsrichtung 20 sowie eine bestimmte radiale Richtung 22, entlang welcher der Korrekturstab im Gabelkopf 6 aufnehmbar ist.

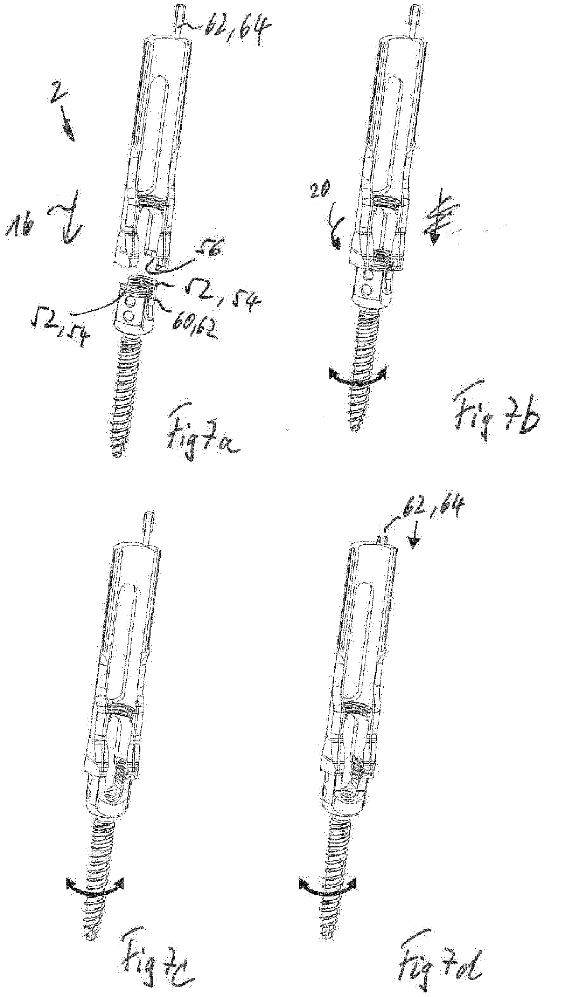

Vor dem Einsetzen und Fixieren eines Korrekturstabs ist es indessen häufig erforderlich, dass an dem Knochen repositionierende Korrekturmaßnahmen durch Einleiten von Kräften und Momenten in den in den Knochen eingeschraubten Ankerschaft ausgeführt werden. Hierzu erweist es sich als vorteilhaft oder gar als erforderlich, dass der Gabelkopf gegenüber dem in den Knochen eingebrachten Schaft des Knochenankers in einer vorm Chirurgen gewünschten Orientierung temporär, d.h. lösbar, festgelegt ist, um währenddessen die erwähnten Korrekturmaßnahmen, vorzugsweise unter Verwendung einer retraktierenden Komponente oder Retraktorvorrichtung ausführen zu können. Wenn der Schaft 4 des Knochenankers 2 in den Knochen eingebracht ist, so ist zunächst der Gabelkopf 6 bezüglich des Schafts 4 im beispielhaft dargestellten Fall polyaxial orientierbar, was in einigen Figuren durch einen Doppelpfeil im Bereich des Schafts 4 angedeutet ist. Zum temporären Festlegen der Orientierung des Gabelkopfs 6 gegenüber dem Schaft 4 ist ein im Einzelnen in Figuren 7a-c dargestelltes Klemmstück 26 vorgesehen, welches durch die Zugangsöffnung 10 hindurch dem Gabelkopf 6 zuführbar ist.

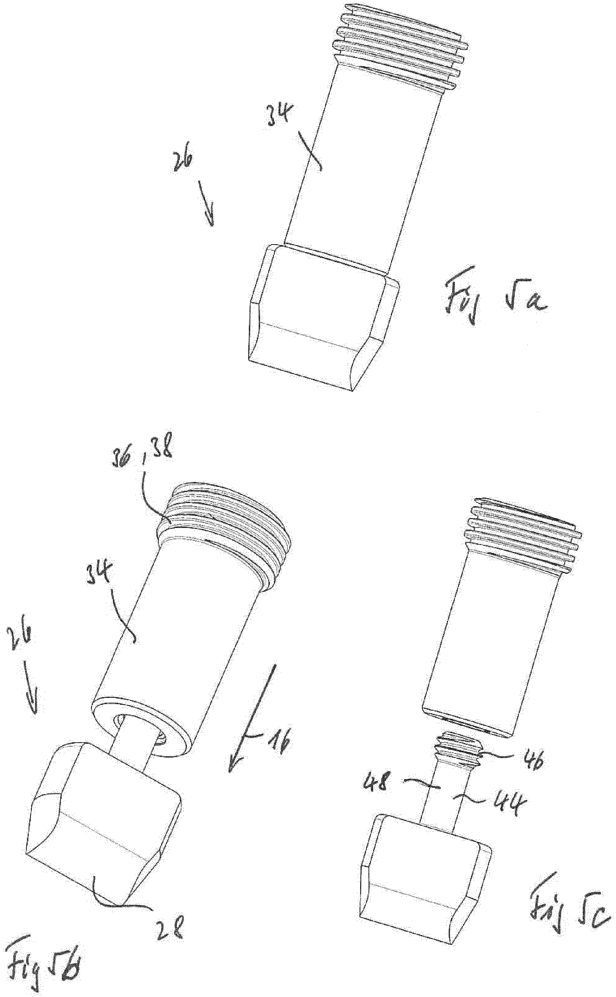

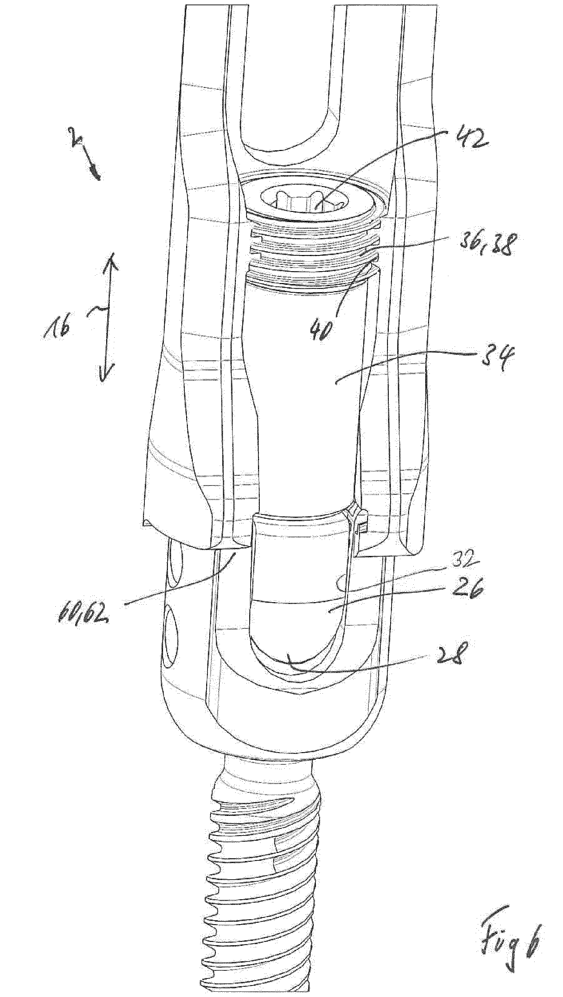

Das durch die Zugangsöffnung 10 hindurchdurchgeführte Klemmstück 26 stützt sich mit einem Auflagerabschnitt 28 gegen einen Kopf des im Gabelkopf 6 zunächst polyaxial verschwenkbar gelagerten Ankerschafts 4 ab, wobei im beispielhaft dargestellten Fall noch ein Zwischenstück 30 zwischen dem Kopf des Schafts 4 und dem Auflagerabschnitt 28 des Klemmstücks 26 in den Gabelkopf 6 eingesetzt ist. Das Zwischenstück 30 ist einerseits komplementär zu dem Kopf des Schafts 4 und andererseits komplementär zu dem Auflagerabschnitt 28 des Klemmstücks 26 ausgebildet. Der Gabelkopf 6 und dessen Schenkel begrenzen eine Kernöffnung 32 mit einem Kerndurchmesser, in welche das Zwischenstück 30 und das Klemmstück 26 einsetzbar sind. Das Klemmstück 26 erstreckt sich aber über die Kernöffnung 32 in der bestimmten Richtung 22 nach außen und ist somit gegen die Schenkel des Gabelkopf abgestützt und daher in der Umfangsrichtung 20 im Gabelkopf 6 unverdrehbar. Das in den Gabelkopf 6 eingesetzte Klemmstück 26 ist weiter durch ein Stellmittel 34 in der axialen Längsrichtung 16 mit einer temporären Stellkraft beaufschlagbar, die dadurch erzeugt wird, dass sich das Stellmittel 34 in der axialen Längsrichtung 16 gegen die Verlängerungsvorrichtung 8, d.h. gegen das Schalenteil 12 abstützt. Hierzu weist das Stellmittel 34 einen Gewindeträgerabschnitt 36 mit einem Außengewinde 38 und das Schalenteil 12 ein Innengewinde 40 auf, in welches das Außengewinde 38 des Gewindeträgerabschnitts 36 einschraubbar ist, so dass hierdurch in der axialen Längsrichtung 16 die temporäre Stellkraft über das Klemmstück 26 auf den Schaft 4 ausübbar ist, um die Orientierung des Gabelkopfs 6 gegenüber dem Schaft 4 temporär und lösbar zu fixieren. Diesen temporär fixierten Klemmzustand zeigt Figur 6. Man erkennt, dass das Stellmittel 34 am Ende des Gewindeträgerabschnitts 36 eine Werkzeugansetzstelle aufweist, so dass das Stellmittel 34 mittels eines durch die Zugangsöffnung durchgeführten Instrumentenschafts 10 mit seinem Gewindeträgerabschnitt 36 in die dargestellte Klemmposition in das Innengewinde 40 eingeschraubt werden kann. Man erkennt weiter aus den Figuren 5a-c, dass das Stellmittel 34 gegenüber dem Klemmstück 26 beschränkt axial verschieblich ist, indem es auf einem in der Längsrichtung 16 erstreckten Stift 44 sitzt, welcher fest mit dem Klemmstück 16 ausgebildet ist. Am freien Ende des Stifts 44 ist ein kurzes Außengewinde 46 ausgebildet, auf welches ein im Inneren des Stellmittels 34 ausgebildetes ebenfalls kurzes Innengewinde aufschraubbar ist, indem das Stellmittel 34 auf den Stift 44 aufgedreht wird, und zwar so lange, bis das Innengewinde innerhalb des Stellmittels 34 das kurze Außengewinde 46 verlassen hat. Das Stellmittel 34 ist dann auf der Länge eines gewindelosen Abschnitts 48 des Stifts 44 gegenüber dem Klemmstück 26 hin- und herbewegbar in der axialen Längsrichtung 16 und dabei unverlierbar an dem Klemmstück 26 gehalten. Es kann somit zusammen mit dem Klemmstück 26 durch die Zugangsöffnung 10 zugeführt und wieder entnommen werden.

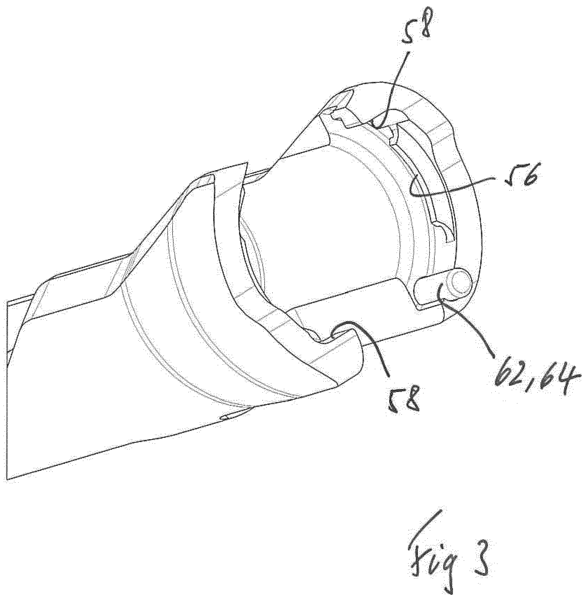

Damit das Klemmstück 26 wie vorstehend beschrieben zum lösbaren Fixieren der Orientierung des Gabelkopfs 6 gegenüber dem in den Knochen eingeschraubten Schaft 4 zugeführt und fixiert werden kann ist es erforderlich, dass zuvor die Verlängerungsvorrichtung 8, die im beispielhaften Fall von dem einzigen Schalenteil 12 gebildet ist, am Gabelkopf 6 winkelstabil fixiert wird. Dies ist in Figuren 7 a-d dargestellt. Hierfür weist der Gabelkopf 6 an der Außenseite seiner beiden Schenkel 7 einen konzentrisch in der Umfangsrichtung 20 erstreckten hintergreifbaren Bereich 52 in Form einer im Querschnitt hakenartig anmutenden Nut 54 auf. Das Schalenteil 12 weist einen hierzu komplementär ausgebildeten hintergreifenden Bereich 56. Die Bereiche 52 und 56 sind dabei derart ausgebildet, dass das Schalenteil in der axialen Längsrichtung 16 an den Gabelkopf herangeführt wird und dann in der Umfangsrichtung 20 in eine Hintergriffsstellung drehbar ist, in der der hintergreifende Bereich 56 des Schalenteils 12 in den \ hintergreifbaren Bereich 52 des Gabelkopf 6 so eingreift, dass ein formschlüssiger Hintergriff bezüglich der axialen Längsrichtung 16 und auch bezüglich der radialen Richtung 18 gegeben ist, so dass das Schalenteil 12 winkelstabil mit dem Gabelkopf verbunden ist. Wie man des Weiteren aus Figur 3 erkennt weist jeder Befestigungsabschnitt 14 des Schalenteils 12 einen in Umfangsrichtung 20 orientierten Endanschlag 58, wobei die Endanschläge 58 einander diametral gegenüberliegen, so dass das Schalenteil 12 auf den Gabelkopf 6 aufdrehbar ist, bis die Endanschläge 58 an korrespondierenden Anschlägen 60 der Schenkel des Gabelkopfs 6 einliegen. Diese korrespondierenden Anschläge 60 sind von Seitenflanken 62 der Schenkel des Gabelkopfs 6 gebildet. Das Schalenteil 12 ist in der in Figur 6 gezeigten Montageposition am Gabelkopf 6 zudem gegen Zurückdrehen sicherbar. Hierfür ist ein der axialen Längsrichtung 16 erstrecktes Sperrorgan 62 in Form eines Stifts 64 vorgesehen, welcher in dem Schalenteil 12 in der axialen Längsrichtung 16 verschieblich aufgenommen ist. Es ist vom oberen freien Ende des Schalenteils 12 her zugänglich und lässt sich mit seinem freien Ende bis über den hintergreifenden Bereich 56 eines unteren Befestigungsabschnitts 14 des Schalenteils 12 verschieben, so dass es dort gegen eine Seitenflanke eines Schenkels 7 des Gabelkopfs 6 anliegt und das Schalenteil 12 so gegen Rückdrehen sichert (Fig 6).

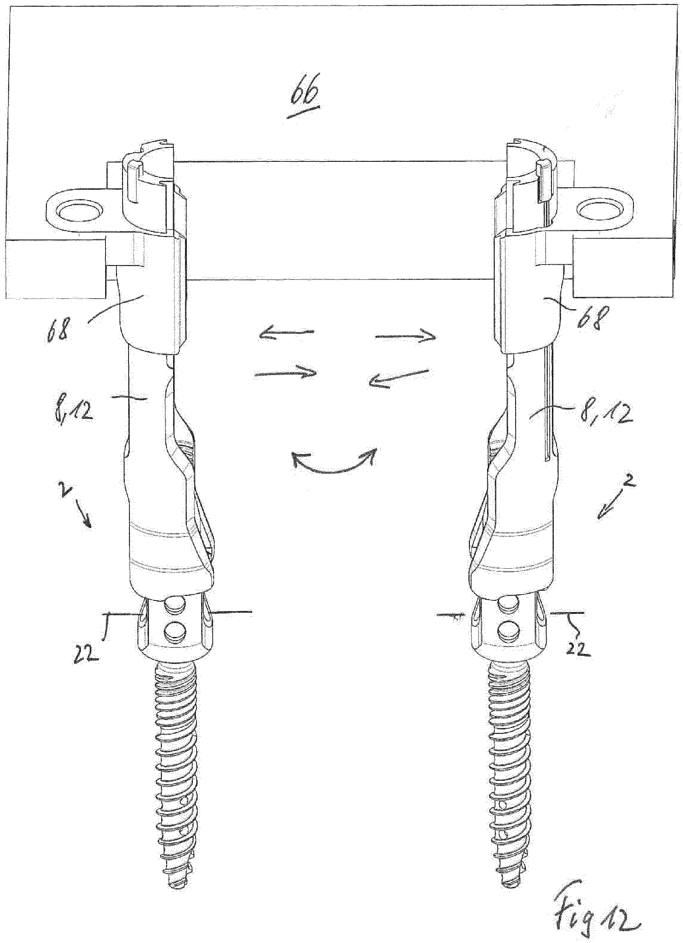

Die eingangs erwähnten Korrekturmaßnahmen werden mittels einer retraktierenden Komponente 66 ausgeführt, die nur schematisch in Figur 12 angedeutet ist. Einige hier infrage kommenden Korrekturmaßnahmen, insbesondere Kompression, Distraktion, Lordosierung, Kyphosierung sind in Figur 12 durch Pfeile bei der retraktierenden Komponente 66 angedeutet. Hierfür wird die retraktierende Komponente 66 zur Einleitung von Kräften und Momenten auf den Knochen mittels eines jeweiligen Verbindungsteils 68 mit dem jeweiligen Schalenteil 12 der Verlängerungsvorrichtung 8 verbunden.

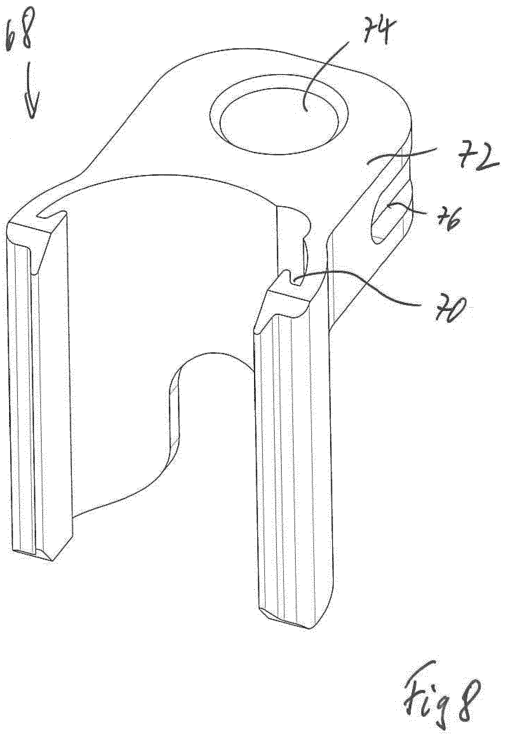

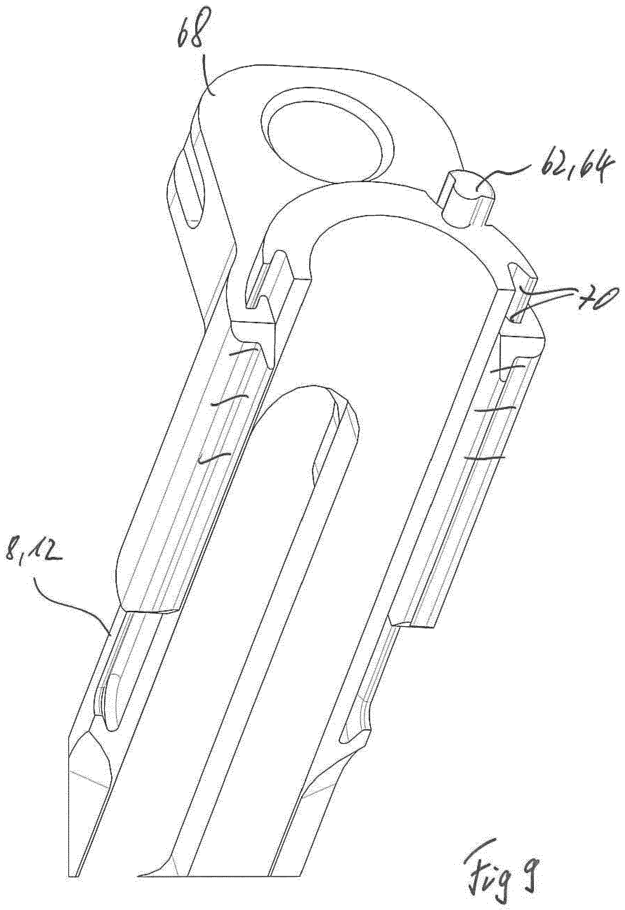

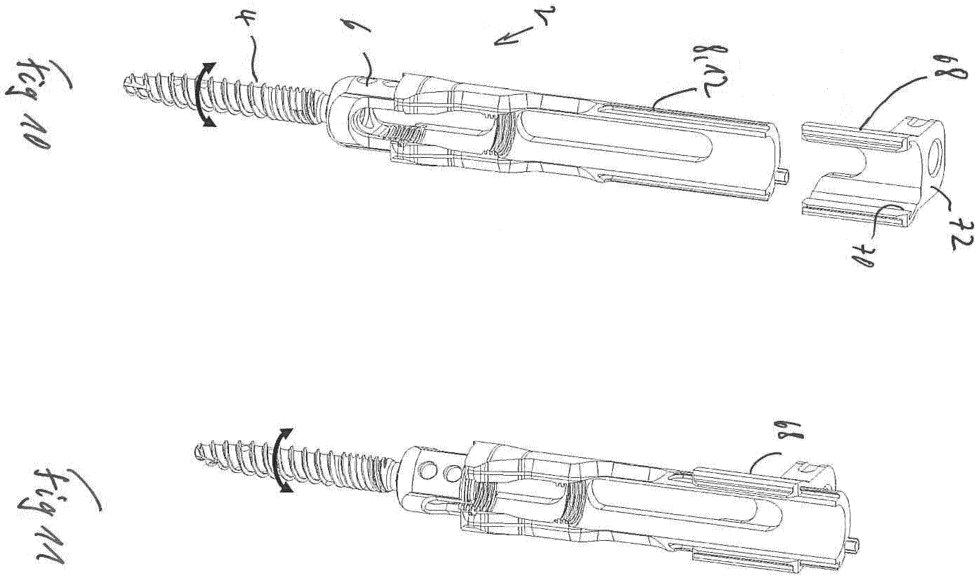

Dieses Verbindungsteil 68 ist in den Figuren 8 und 9 dargestellt, und Figuren 10, 11 zeigen das Aufschieben des Verbindungsteils 68 in der axialen Längsrichtung 16 auf die Verlängerungsvorrichtung 8. Hierfür weist das Verbindungsteil 68 und auch das Schalenteil 12 miteinander zusammenwirkende Längsführungsmittel 70 auf. Das Verbindungsteil 68 ist ebenfalls ungefähr halbschalenförmig und komplementär zu dem Schalenteil 12 ausgebildet und weist einen nach radial außen erstreckten Befestigungsabschnitt 72 auf, der beispielhaft eine Eingriffsöffnung 74 aufweist, in welche die retraktierende Komponente 66 eingreifen kann, so dass eine starre Betriebsverbindung zwischen retraktierender Komponente 66 und dem Verbindungsteil 68 herstellbar ist. An dem Befestigungsabschnitt 72 des Verbindungsteils 68 sind seitlich randoffene Nuten 76 ausgebildet, in welche ein nicht dargestelltes Sicherungsmittel zur Verhinderung des unbeabsichtigten Ablösens der retraktierenden Komponente 66 eingreifen kann.

Nach dem Verbinden der jeweiligen Verlängerungsvorrichtung 8 mit der retraktierenden Komponente 66 und vor dem Einleiten von Kräften und Momenten mittels der retraktierenden Komponente 66 wird das vorausgehend beschriebene Klemmstück 26 mit dem daran unverlierbar gehaltenen beweglichen Stellmittel 34 durch die Zugangsöffnung 10 zugeführt und mittels eines Schraubendreher-Instruments in die in der Figur 6 dargestellte Fixierstellung gebracht, in der unter Erzeugung der axialen Stellkraft das Klemmstück 26 sich einerseits über die Gewindeverbindung gegen das Schalenteil 12 und andererseits über das Zwischenstück gegen den Kopf des Schafts 4 derart abstützt, dass die Verlängerungsvorrichtung 8 und der Gabelkopf 6 in einer vom Chirurgen intendierten Winkelstellung gegenüber dem Schaft 4 fixiert ist. In dieser fixierten Stellung werden dann über die retraktierende Komponente 66 Korrekturmaßnahmen an den Knochen oder Wirbelkörpern ausgeführt. Nach Finalisierung dieser Korrekturmaßnahmen wird die temporäre Fixierung aufgelöst, indem der Gewindeträgerabschnitt 36 aus dem Innengewinde 40 herausgedreht und zusammen mit dem dabei unverlierbar gehaltenen Klemmstück 26 wieder aus der Zugangsöffnung 10 entnommen wird. Im Anschluss daran kann ein nicht dargestellter Korrekturstab in an sich bekannter Weise ebenfalls durch die Zugangsöffnung 10 hindurch zugeführt und und dann durch den Trennschlitz 15 hindurch geneigt werden, so dass der Korrekturstab zu einem benachbarten Knochenanker geführt und dort zwischen den Schenkeln des Gabelkopfs 6 im Gabelkopf dauerhaft fixiert werden kann. Letzteres geschieht wiederum in an sich bekannter Weise durch Einschrauben einer nicht dargestellten Madenschraube in ein an der Innenseite der Schenkel 7 des Gabelkopfs 6 ausgebildetes Innengewinde. Der Korrekturstab ist dann im Gabelkopf 6 in der eingangs genannten bestimmten Richtung 22 erstreckt und dort dauerhaft geklemmt und hierdurch fixiert.

## Patentansprüche

1. Knochenanker (2), insbesondere Knochenschraube, insbesondere Pedikelschraube, insbesondere für die minimalinvasive Wirbelsäulenchirurgie,
wobei der Knochenanker (2) einen in einen Knochen einsetzbaren Schaft (4) und einen gegenüber dem Schaft polyaxial orientierbaren Gabelkopf (6) mit zwei Schenkeln (7) zur Aufnahme eines Korrekturstabs umfasst, wobei der Gabelkopf (6) eine axiale Längsrichtung (16), eine hierzu konzentrische Umfangsrichtung (20) und eine radiale Richtung (18) definiert, wobei der Gabelkopf (6) mit seinen Schenkeln (7) eine Kernöffnung (32) mit einem Kerndurchmesser begrenzt und eine bestimmte radiale Richtung (22) definiert, entlang welcher der Korrekturstab im Gabelkopf (6) aufnehmbar ist,
mit einer Verlängerungsvorrichtung (8), die eine Zugangsöffnung (10) zu dem Gabelkopf (6) und zu dem Schaft (4) bildet und die winkelstabil an dem Gabelkopf (6) lösbar befestigbar ist,
wobei eine vom Chirurgen gewünschte Orientierung des Gabelkopfs (6) gegenüber dem Schaft (4) temporär, das heißt lösbar festlegbar ist, um währenddessen Korrekturmaßnahmen bei dem Knochen ausführen zu können,
**dadurch gekennzeichnet,**
**dass** der Knochenanker (2) weiterhin ein Klemmstück (26) umfasst, das zum temporären Festlegen der Orientierung des Gabelkopfs (6) gegenüber dem Schaft (4) durch die Zugangsöffnung (10) hindurch dem Gabelkopf (6) zuführbar ist und dort unmittelbar oder mittelbar gegen den Schaft (4) klemmbar ist, indem auf das Klemmstück (26) eine temporäre Stellkraft in der axialen Längsrichtung (16) ausübbar ist, und dass der Knochenanker (2) weiterhin ein Stellmittel (34) umfasst und dass die temporäre Stellkraft in der axialen Längsrichtung (16) durch Abstützung des Stellmittels (34) gegen die Verlängerungsvorrichtung (8) erzeugbar und in das Klemmstück (26) einleitbar ist,
und **dass** sich das Klemmstück (26) zwischen den Schenkeln (7) des Gabelkopfs (6) in der bestimmten radialen Richtung (22) über die Kernöffnung (32) nach außen erstreckt und somit gegen die Schenkel (7) des Gabelkopfs (6) abgestützt und in Umfangsrichtung (20) unverdrehbar ist.

2. Knochenanker (2) nach Anspruch 1, **dadurch gekennzeichnet, dass** das Klemmstück (26) einen Auflagerabschnitt (28) aufweist, mit dem es mittelbar oder unmittelbar gegen den Schaft (4) anlegbar ist und dass sich das Klemmstück (26) dabei insbesondere mit diesem Auflagerabschnitt (28) in der bestimmten radialen Richtung (22) über die Kernöffnung (32) nach außen erstreckt.

3. Knochenanker (2) nach Anspruch 1 oder 2, **dadurch gekennzeichnet, dass** das Stellmittel (34) einen Gewindeträgerabschnitt (36) mit einem Außengewinde (38) aufweist, und dass die Verlängerungsvorrichtung (8) ein Innengewinde (40) aufweist, in welches das Außengewinde (38) des Gewindeträgerabschnitts (36) einschraubbar ist, so dass hierdurch die temporäre Stellkraft in der axialen Längsrichtung (16) über das Klemmstück (26) auf den Schaft (4) ausübbar ist, um die Orientierung des Gabelkopfs (6) gegenüber dem Schaft (4) temporär und lösbar zu fixieren.

4. Knochenanker (2) nach Anspruch 1, 2 oder 3, **dadurch gekennzeichnet, dass** das Stellmittel (34), insbesondere der Gewindeträgerabschnitt (36), unverlierbar mit dem Klemmstück (26) angeordnet ist.

5. Knochenanker (2) nach einem oder mehreren der vorstehenden Ansprüche, **dadurch gekennzeichnet, dass** das Stellmittel (34), insbesondere der Gewindeträgerabschnitt (36), axial verschieblich an dem Klemmstück (26) geführt und vorzugsweise drehbar an dem Klemmstück (26) gehalten ist.

6. Knochenanker (2) nach einem oder mehreren der vorstehenden Ansprüche 3 - 5, **dadurch gekennzeichnet, dass** der Gewindeträgerabschnitt (36) auf einem in der axialen Längsrichtung (16) erstreckten und an dem Klemmstück (26), insbesondere an dem Auflagerabschnitt (28) des Klemmstücks (26), gehaltenen Stift (44) beschränkt verschieblich und drehbar geführt ist.

7. Knochenanker (2) nach einem oder mehreren der vorstehenden Ansprüche, **dadurch gekennzeichnet, dass** in den Gabelkopf (6) ein Zwischenstück (30) eingesetzt ist, welches zwischen einem Kopf des Schafts (4) und einem in den Gabelkopf (6) einlegbaren und dort dauerhaft fixierbaren Korrekturstab bzw. dem Auflagerabschnitt (28) des Klemmstücks (26) angeordnet ist, und dass der Auflagerabschnitt (28) des Klemmstücks (26) auf seiner dem Zwischenstück (30) zugewandten Seite komplementär zu dem Zwischenstück (30) und insbesondere stabrund ausgebildet ist.

8. Knochenanker (2) nach einem oder mehreren der vorstehenden Ansprüche, **dadurch gekennzeichnet, dass** die Verlängerungsvorrichtung (8) als starres, verwindungssteifes Hülsenteil oder Schalenteil (12) ausgebildet ist.

9. Knochenanker (2) nach Anspruch 8, **dadurch gekennzeichnet, dass** die Verlängerungsvorrichtung (8) ein einziges starres, verwindungssteifes Hülsenteil oder Schalenteil (12) umfasst, welches an seinem dem Gabelkopf (6) zugewandten Ende zwei voneinander beabstandete und durch einen in der axialen Längsrichtung ausmündenden Trennschlitz (15) voneinander getrennte Befestigungsabschnitte (14) aufweist, mittels derer das Hülsenteil oder Schalenteil (12) an den Schenkeln (7) des Gabelkopfs (6) lösbar festlegbar ist.

10. Knochenanker (2) nach einem oder mehreren der vorstehenden Ansprüche, **dadurch gekennzeichnet, dass** das Innengewinde (40) an der Verlängerungsvorrichtung (8) eine Erstreckung in der Umfangsrichtung (20) von wenigstens 180°, insbesondere von wenigstens 181°, insbesondere von wenigstens 182°, insbesondere von wenigstens 185° und insbesondere von höchstens 200°, insbesondere von höchstens 195°, insbesondere von höchstens 190° aufweist.

11. Knochenanker (2) nach einem oder mehreren der vorstehenden Ansprüche, **dadurch gekennzeichnet, dass** die Verlängerungsvorrichtung (8) in eine Hintergriffsstellung am Gabelkopf (6) drehbar ist.

12. Knochenanker (2) nach Anspruch 11, **dadurch gekennzeichnet, dass** die Verlängerungsvorrichtung (8) in der Hintergriffsstellung gegen Rückdrehen sicherbar ist.

13. Knochenanker (2) nach Anspruch 12, **gekennzeichnet durch** ein Sicherungsmittel in Form eines in der axialen Längsrichtung (16) verschieblichen Sperrorgans (62), insbesondere eines Stifts (64), das in der Verlängerungsvorrichtung (8) geführt ist und sich in Umfangsrichtung (20) gegen einen Schenkel (7) des Gabelkopfs (6) abstützt und hierdurch ein unbeabsichtigtes Rückdrehen der Verlängerungsvorrichtung (8) verhindert.

14. Knochenanker (2) nach Anspruch 13, **dadurch gekennzeichnet, dass** sich das Sperrorgan (62) in der axialen Längsrichtung (16) bis zu einem vom Gabelkopf (6) abgewandten Ende der Verlängerungsvorrichtung (8) erstreckt und dort zur Ausführung einer Stellbewegung zugänglich ist.

15. Knochenanker (2) nach einem oder mehreren der vorstehenden Ansprüche, **dadurch gekennzeichnet, dass** die Verlängerungsvorrichtung (8) in der Hintergriffsstellung gegen einen in der Umfangsrichtung (20) wirkenden Anschlag (60) am Gabelkopf anlegbar ist.

16. Knochenanker (2) nach einem oder mehreren der vorstehenden Ansprüche, **dadurch gekennzeichnet**, **gekennzeichnet durch** ein Verbindungsteil (68) als Schnittstelle zwischen der am Gabelkopf (6) festgelegten Verlängerungsvorrichtung (8) und einer retraktierenden Komponente (66), wobei das Verbindungsteil (68) lösbar an der Verlängerungsvorrichtung (8) anordenbar ist.

17. Knochenanker (2) nach Anspruch 16, **dadurch gekennzeichnet, dass** das Verbindungsteil (68) in der axialen Längsrichtung (16) lösbar auf die Verlängerungsvorrichtung (8) aufschiebbar oder jedenfalls an der Verlängerungsvorrichtung (8) verschieblich ist und hierfür ein in der axialen Längsrichtung (16) erstrecktes Längsführungsmittel (70) an der Verlängerungsvorrichtung (8) und an dem Verbindungsteil (68) vorgesehen ist.

18. Knochenanker (2) nach Anspruch 16 oder 17, **dadurch gekennzeichnet, dass** das Längsführungsmittel (70) formschlüssig wirkend ist und insbesondere nut- und federartig, insbesondere wenigstens halbseitig schwalbenschwanzförmig, ausgebildet ist.

19. Knochenanker (2) nach Anspruch 16 oder 17 oder 18, **dadurch gekennzeichnet, dass** das Verbindungsteil (68) einen schalenförmigen und komplementär zu der Verlängerungsvorrichtung (8) ausgebildeten Abschnitt aufweist.

20. Knochenanker (2) nach einem der Ansprüche 16 - 19, **dadurch gekennzeichnet, dass** das Verbindungsteil (68) einen quer zur axialen Längsrichtung (16) nach außen erstreckten Befestigungsabschnitt (72) zur lösbaren formschlüssigen Verbindung und Fixierung mit einer retraktierenden Komponente (66) aufweist.

## Claims

1. Bone anchor (2), in particular a bone screw, in particular a pedicle screw, in particular for minimally invasive spinal surgery,
wherein the bone anchor (2) comprises a shaft (4) which can be inserted into a bone and a fork head (6) which can be oriented multi-axially with respect to the shaft and has two legs (7) for receiving a correction rod, wherein the fork head (6) defines an axial longitudinal direction (16), a circumferential direction (20) concentric therewith, and a radial direction (18), wherein the fork head (6) with its legs (7) delimits a core opening (32) with a core diameter and defines a specific radial direction (22) along which the correction rod can be received in the fork head (6),
having an extension device (8) which forms an access opening (10) to the fork head (6) and to the shaft (4) and which can be detachably fastened to the fork head (6) in an angularly stable manner,
wherein an orientation of the fork head (6) with respect to the shaft (4) intended by the surgeon can be temporarily, i.e., detachably, fixed in order to be enable carrying out corrective measures on the bone during this time,
**characterized in that**, the bone anchor (2) comprising a a clamping piece (26) for temporarily fixing the orientation of the fork head (6) relative to the shaft (4), which can be fed through the access opening (10) to the fork head (6) and can be clamped there directly or indirectly against the shaft (4) by exerting a temporary actuating force on the clamping piece (26) in the axial longitudinal direction (16), and **in that** the bone anchor (2) further comprising an actuating means (34) and **in that** the temporary actuating force can be generated in the axial longitudinal direction (16) by bracing the actuating means (34) against the extension device (8), and can be introduced into the clamping piece (26),
and **in that** the clamping piece (26) extends outwards between the legs (7) of the fork head (6) in the specific radial direction (22) via the core opening (32) and is therefore braced against the legs (7) of the fork head (6) and cannot be rotated in the circumferential direction (20).

2. Bone anchor (2) according to claim 1, **characterized in that** the clamping piece (26) has a contact portion (28) with which it can be placed directly or indirectly against the shaft (4), and that the clamping piece (26) extends outwards in the specific radial direction (22) via the core opening (32), in particular with this contact portion (28).

3. Bone anchor (2) according to claim 1 or 2, **characterized in that** the actuating means (34) has a thread carrier portion (36) with an external thread (38), and **in that** the extension device (8) has an internal thread (40) into which the external thread (38) of the thread carrier portion (36) can be screwed, such that the temporary actuating force can be exerted in the axial longitudinal direction (16) onto the shaft (4) via the clamping piece (26) in order to temporarily and releasably fix the orientation of the fork head (6) relative to the shaft (4).

4. Bone anchor (2) according to claim 1, 2, or 3, **characterized in that** the actuating means (34), in particular the thread carrier portion (36), is arranged captively with the clamping piece (26).

5. Bone anchor (2) according to one or more of the preceding claims, **characterized in that** the actuating means (34), in particular the thread carrier portion (36), is guided axially displaceably on the clamping piece (26) and is preferably held in a manner allowing rotation on the clamping piece (26).

6. Bone anchor (2) according to one or more of the preceding claims 3 - 5, **characterized in that** the thread carrier portion (36) is guided in a limitedly displaceable and rotatable manner on a pin (44) which extends in the axial longitudinal direction (16) and which is held on the clamping piece (26), in particular on the contact portion (28) of the clamping piece (26).

7. Bone anchor (2) according to one or more of the preceding claims, **characterized in that** an intermediate piece (30) is inserted into the fork head (6), which intermediate piece is arranged between a head of the shaft (4) and a correction rod that can be inserted into the fork head (6) and permanently fixed there or the contact portion (28) of the clamping piece (26), and **in that** the contact portion (28) of the clamping piece (26) is designed on its side facing the intermediate piece (30) to be complementary to the intermediate piece (30) and in particular to be round like a rod.

8. Bone anchor (2) according to one or more of the preceding claims, **characterized in that** the extension device (8) is designed as a rigid, torsion-resistant sleeve part or shell part (12).

9. Bone anchor (2) according to claim 8, **characterized in that** the extension device (8) comprises a single rigid, torsion-resistant sleeve part or shell part (12) which, at its end facing the fork head (6), has two fastening portions (14) which are spaced apart from one another and separated from one another by a separating slot (15) opening out in the axial longitudinal direction, and by means of which the sleeve part or shell part (12) can be releasably fixed to the legs (7) of the fork head (6).

10. Bone anchor (2) according to one or more of the preceding claims, **characterized in that** the internal thread (40) on the extension device (8) has an extension in the circumferential direction (20) of at least 180°, in particular of at least 181°, in particular of at least 182°, in particular of at least 185° and in particular of at most 200°, in particular of at most 195°, in particular of at most 190°.

11. Bone anchor (2) according to one or more of the preceding claims, **characterized in that** the extension device (8) can be rotated into a position engaging behind the fork head (6).

12. Bone anchor (2) according to claim 11, **characterized in that** the extension device (8) can be secured in the rear engagement position against rotating back.

13. Bone anchor (2) according to claim 12, **characterized by** a securing means in the form of a blocking member (62) displaceable in the axial longitudinal direction (16), in particular a pin (64), which is guided in the extension device (8) and is braced in the circumferential direction (20) against a leg (7) of the fork head (6) and thereby prevents an unintentional backward rotation of the extension device (8).

14. Bone anchor (2) according to claim 13, **characterized in that** the blocking member (62) extends in the axial longitudinal direction (16) to an end, remote from the fork head (6), of the extension device (8) and is there accessible for carrying out an actuating movement.

15. Bone anchor (2) according to one or more of the preceding claims, **characterized in that** the extension device (8) in the rear engagement position can be placed against a stop (60), acting in the circumferential direction (20), on the fork head.

16. Bone anchor (2) according to one or more of the preceding claims, **characterized by** a connecting part (68) as an interface between the extension device (8), fixed to the fork head (6), and a retracting component (66), wherein the connecting part (68) can be detachably arranged on the extension device (8).

17. Bone anchor (2) according to claim 16, **characterized in that** the connecting part (68) can be releasably pushed onto the extension device (8) in the axial longitudinal direction (16) or in any case is displaceable on the extension device (8), and for this purpose a longitudinal guide means (70) extending in the axial longitudinal direction (16) is provided on the extension device (8) and on the connecting part (68).

18. Bone anchor (2) according to claim 16 or 17, **characterized in that** the longitudinal guide means (70) acts with a positive fit, and in particular has a tongue-and-groove design, in particular is dovetail-shaped on at least one of two sides.

19. Bone anchor (2) according to claim 16 or 17 or 18, **characterized in that** the connecting part (68) has a portion which is shell-shaped and is complementary to the extension device (8).

20. Bone anchor (2) according to one of claims 16 - 19, **characterized in that** the connecting part (68) has a fastening portion (72), extending outwards transversely to the axial longitudinal direction (16), to create a releasable, positive connection and fixation to a retracting component (66).

## Revendications

1. Ancre à os (2), en particulier vis à os, en particulier vis pédiculaire, en particulier pour la chirurgie rachidienne mini-invasive,
dans lequel ledit ancre à os (2) comprend une tige (4) insérable dans un os et une tête fourchue (6) orientable polyaxialement par rapport à la tige et munie de deux branches (7) destinées à recevoir une barre de correction, dans lequel ladite tête fourchue (6) définit une direction longitudinale axiale (16), une direction circonférentielle (20) concentrique à celle-ci et une direction radiale (18), dans lequel ladite tête fourchue (6) délimite avec ses branches (7) une ouverture centrale (32) de diamètre de noyau et définit une direction radiale spécifique (22) le long de laquelle la barre de correction peut être reçue dans la tête fourchue (6),
avec un dispositif d'extension (8) qui forme une ouverture d'accès (10) à la tête fourchue (6) et à la tige (4) et qui peut être fixé de manière amovible et angulairement stable à la tête fourchue (6),
dans lequel une orientation de la tête fourchue (6) par rapport à la tige (4), qui est souhaitée par le chirurgien, peut être fixée temporairement, c'est-à-dire de manière amovible, afin de pouvoir réaliser des mesures correctives sur l'os pendant cela,
**caractérisé par le fait**
**que** l'ancre à os (2) comprend en outre une pièce de serrage (26) qui, pour fixer temporairement l'orientation de la tête de fourche (6) par rapport à la tige (4), peut être amenée à la tête fourchue (6) par ladite ouverture d'accès (10) et y être serrée directement ou indirectement contre la tige (4) en exerçant une force de réglage temporaire sur la pièce de serrage (26) dans la direction axiale longitudinale (16), et
**que** l'ancre à os (2) comprend en outre un moyen de réglage (34) et que la force de réglage temporaire dans la direction axiale longitudinale (16) peut être générée en appuyant ledit moyen de réglage (34) contre le dispositif d'extension (8) et peut être appliquée à la pièce de serrage (26),
et **que** la pièce de serrage (26) s'étend entre les branches (7) de la tête fourchue (6) dans la direction radiale spécifique (22) par l'orifice central (32) vers l'extérieur et est ainsi appuyée contre les branches (7) de la tête fourchue (6) et ne peut pas tourner dans la direction circonférentielle (20).

2. Ancre à os (2) selon la revendication 1, **caractérisé par le fait que** la pièce de serrage (26) comprend une section d'appui (28) par l'intermédiaire de laquelle elle peut être appliquée directement ou indirectement contre la tige (4) et que la pièce de serrage (26) s'étend, en particulier avec cette section d'appui (28), dans la direction radiale spécifique (22) par l'ouverture centrale (32) vers l'extérieur.

3. Ancre à os (2) selon la revendication 1 ou 2, **caractérisé par le fait que** le moyen de réglage (34) comprend une section de support filetée (36) dotée d'un filetage extérieur (38), et que le dispositif d'extension (8) comprend un filetage intérieur (40) dans lequel le filetage extérieur (38) de la section de support filetée (36) peut être vissé, permettant ainsi d'exercer la force de réglage temporaire dans la direction axiale longitudinale (16) sur la tige (4) via la pièce de serrage (26) afin de fixer temporairement et de manière amovible l'orientation de la tête fourchue (6) par rapport à la tige (4).

4. Ancre à os (2) selon la revendication 1, 2 ou 3, **caractérisé par le fait que** le moyen de réglage (34), en particulier la section de support filetée (36), est disposé de manière imperdable avec la pièce de serrage (26).

5. Ancre à os (2) selon une ou plusieurs des revendications précédentes, **caractérisé par le fait que** le moyen de réglage (34), en particulier la section de support filetée (36), est guidé de manière axialement déplaçable sur la pièce de serrage (26) et est de préférence maintenu de manière rotative sur la pièce de serrage (26).

6. Ancre à os (2) selon une ou plusieurs des revendications 3 à 5 précédentes, **caractérisé par le fait que** la section de support filetée (36) est guidée de manière à être pouvoir être déplacée et tournée de façon limitée sur une goupille (44) s'étendant dans la direction longitudinale axiale (16) et maintenue sur la pièce de serrage (26), en particulier sur la section d'appui (28) de la pièce de serrage (26).

7. Ancre à os (2) selon une ou plusieurs des revendications précédentes, **caractérisé par le fait qu'**une pièce intermédiaire (30) est insérée dans la tête fourchue (6), laquelle est disposée entre une tête de la tige (4) et une barre de correction apte à être insérée dans la tête fourchue (6) et y être fixée de manière permanente, ou bien la section d'appui (28) de la pièce de serrage (26), et que la section d'appui (28) de la pièce de serrage (26) est réalisée, sur sa face montrant vers la pièce intermédiaire (30), de façon complémentaire à la pièce intermédiaire (30) et en particulier de manière ronde comme une barre.

8. Ancre à os (2) selon une ou plusieurs des revendications précédentes, **caractérisé par le fait que** le dispositif d'extension (8) est conçu en tant que partie de manchon ou partie de coque (12) rigide et résistante à la torsion.

9. Ancre à os (2) selon la revendication 8, **caractérisé par le fait que** le dispositif d'extension (8) comprend une seule partie de manchon ou de coque (12) rigide et résistante à la torsion qui présente, à son extrémité montrant vers la tête fourchue (6), deux sections de fixation (14) qui sont espacées l'une de l'autre et séparées l'une de l'autre par une fente de séparation (15) débouchant dans la direction longitudinale axiale et au moyen desquelles la partie de manchon ou de coque (12) peut être fixée de manière amovible aux branches (7) de la tête fourchue (6).

10. Ancre à os (2) selon une ou plusieurs des revendications précédentes, **caractérisé par le fait que** le filetage interne (40) sur le dispositif d'extension (8) présente une extension dans la direction circonférentielle (20) d'au moins 180°, en particulier d'au moins 181°, en particulier d'au moins 182°, en particulier d'au moins 185°, et en particulier de 200° tout au plus, en particulier de 195° tout au plus, en particulier de 190° tout au plus.

11. Ancre à os (2) selon une ou plusieurs des revendications précédentes, **caractérisé par le fait que** le dispositif d'extension (8) peut être tourné dans une position de prise de derrière sur la tête fourchue (6).

12. Ancre à os (2) selon la revendication 11, **caractérisé par le fait que** le dispositif d'extension (8) peut être bloqué en rotation de retour dans la position de prise de derrière.

13. Ancre à os (2) selon la revendication 12, **caractérisé par** un moyen de sécurité sous forme d'un organe de blocage (62), en particulier d'une goupille (64), déplaçable dans la direction longitudinale axiale (16), qui est guidé dans le dispositif d'extension (8) et s'appuie dans la direction circonférentielle (20) contre une branche (7) de la tête fourchue (6), empêchant ainsi toute rotation de retour accidentelle du dispositif d'extension (8).

14. Ancre à os (2) selon la revendication 13, **caractérisé par le fait que** ledit organe de blocage (62) s'étend dans la direction longitudinale axiale (16) jusqu'à une extrémité du dispositif d'extension (8), qui montre dans la direction opposée à la tête fourchue (6) et y est accessible pour effectuer un mouvement de réglage.

15. Ancre à os (2) selon une ou plusieurs des revendications précédentes, **caractérisé par le fait que** le dispositif d'extension (8) peut être appliqué en position de prise de derrière contre une butée (60) située sur la tête de fourche et agissant dans la direction circonférentielle (20).

16. Ancre à os (2) selon une ou plusieurs des revendications précédentes, **caractérisé par** une pièce de liaison (68) servant d'interface entre le dispositif d'extension (8) fixé à la tête fourchue (6) et un composant rétractable (66), dans lequel la pièce de liaison (68) peut être disposée de manière amovible sur le dispositif d'extension (8).

17. Ancre à os (2) selon la revendication 16, **caractérisé par le fait que** la pièce de liaison (68) peut être glissée de manière amovible sur le dispositif d'extension (8) dans la direction axiale longitudinale (16) ou est en tout cas déplaçable sur le dispositif d'extension (8), et, à cette fin, un moyen de guidage longitudinal (70) s'étendant dans la direction axiale longitudinale (16) est prévu sur le dispositif d'extension (8) et sur la pièce de liaison (68).

18. Ancre à os (2) selon la revendication 16 ou 17, **caractérisé par le fait que** le moyen de guidage longitudinal (70) agit à engagement positif et est réalisé en particulier de type à rainure et languette, en particulier en forme de queue d'aronde au moins sur un côté.

19. Ancre à os (2) selon la revendication 16 ou 17 ou 18, **caractérisé par le fait que** la pièce de liaison (68) présente une section en forme de coque et complémentaire au dispositif d'extension (8).

20. Ancre à os (2) selon l'une quelconque des revendications 16 à 19, **caractérisé par le fait que** la pièce de liaison (68) comprend une section de fixation (72) s'étendant transversalement vers l'extérieur par rapport à la direction longitudinale axiale (16) et destinée à une liaison et une fixation à engagement positif amovible avec un composant rétractable (66).
